# EUROPEAN PATENT SPECIFICATION

(11) **EP 3 879 270 B1**
(45) Date of publication and mention of the grant of the patent: **31.07.2024**
(21) Application number: 19881276.0
(22) Date of filing: 08.11.2019
(51) Int. Cl.: G01N 33/543, G01N 33/574, G01N 33/576

(54) **METHOD FOR DISTINGUISHING VIRAL LIVER CANCER FROM VIRAL HEPATITIS OR VIRAL LIVER CIRRHOSIS**
METHODE ZUR UNTERSCHEIDUNG VON VIRALEM LEBERKREBS VON VIRALER HEPATITIS ODER VIRALER LEBERZIRRHOSE
PROCÉDÉ PERMETTANT DE DISTINGUER LE CANCER VIRAL DU FOIE D'UNE HÉPATITE VIRALE OU D'UNE CIRRHOSE VIRALE DU FOIE

(30) Priority: 09.11.2018 JP 2018211673
(43) Date of publication of application: 15.09.2021
(73) Proprietor: Sekisui Medical Co., Ltd., Tokyo 103-0027 (JP); Miyazaki, Toru, Tokyo 1120003 (JP); Social Welfare Organization "Saiseikai" Imperial Gift Foundation Inc., Tokyo 108-0073 (JP)
(72) Inventor: MIYAZAKI, Toru, Tokyo, 112-0003 (JP); YAMAZAKI Tomoko, 3-1, Hongo 7-chome, Bunkyo- ku Tokyo 113-0033 (JP); OKANOUE Takeshi, Suita-shi, Osaka 564-0013 (JP); ASAI Tomohide, Tokyo 103-0027 (JP); KANETSUKI Yuka, Tokyo 103-0027 (JP); HIROTA, Jiro, Tokyo 103-0027 (JP)
(74) Representative: Hoffmann Eitle
(86) International application number: PCT/JP2019/043859
(87) International publication number: WO 2020/096043

(56) References cited:
- WO-A1-2013/162021
- WO-A1-2017/022315
- WO-A1-2017/043617
- KOYAMA NORIYUKI ET AL: "Activation of apoptosis inhibitor of macrophage is a sensitive diagnostic marker for NASH-associated hepatocellular carcinoma", JOURNAL OF GASTROENTERLOGY, SPRINGER JAPAN KK, JP, vol. 53, no. 6, 30 October 2017 (2017-10-30), pages 770 - 779, XP036513196, ISSN: 0944-1174, [retrieved on 20171030], DOI: 10.1007/S00535-017-1398-Y
- SHIMIZU TOMO ET AL: "Hepatocellular carcinoma diagnosis using a novel electrochemiluminescence immunoassay targeting serum IgM-free AIM", CLINICAL JOURNAL OF GASTROENTEROLOGY, SPRINGER JAPAN, JAPAN, vol. 15, no. 1, 4 January 2022 (2022-01-04), pages 41 - 51, XP037695661, ISSN: 1865-7257, [retrieved on 20220104], DOI: 10.1007/S12328-021-01567-4
- SARVARI, J. ET AL.: "Differentially Expressed Proteins in Chronic Active Hepatitis, Cirrhosis, and HCC Related to HGV Infection in Comparison With HBV Infection: A proteomic study", HEPATITIS MONTHLY, vol. 13, no. 7, 3 July 2013 (2013-07-03), XP055706133, Retrieved from the Internet <URL:https://dx.doi.org/10.5812/hepatmon.8351> [retrieved on 20200109]
- GANGADHARAN, B. ET AL.: "Novel Serum Biomarker Candidates for Liver 1-13 Fibrosis in Hepatitis C Patients", CLINICAL CHEMISTRY, vol. 53, no. 10, 2007, pages 1792 - 1799, XP055018487, DOI: 10.1373/clinchem.2007.089144
- MERA, K. ET AL.: "Serum levels of apoptosis inhibitor of macrophage are associated with hepatic fibrosis in patients with chronic hepatitis C", BMC GASTROENTEROLOGY, vol. 14, no. 27, 13 February 2014 (2014-02-13), XP021177479, Retrieved from the Internet <URL:https://bmcgastroenterol.biomedcentral.com/articles/10.1186/1471-23OX-14-27><DOI:10.1186/1471-23OX-14-27> [retrieved on 20200109]

## Description

### TECHNICAL FIELD

The present invention relates to a method for distinguishing viral liver cancer from viral hepatitis or viral liver cirrhosis. The present invention also relates to a diagnostic agent kit for distinguishing viral liver cancer from viral hepatitis or viral liver cirrhosis.

### BACKGROUND ART

Liver cancer means a malignant tumor occurring in the liver. Liver cancer is a disease with a poor prognosis and is the second leading cause of death among malignant neoplasms worldwide. Main causes of liver cancer are persistent hepatitis virus infection, alcoholic liver disease, and non-alcoholic fatty liver disease (NASH) et al. It is considered that liver cancer is caused by persistent infection with hepatitis virus in 70 to 80% of Japanese liver cancer patients, and a biomarker for diagnosing liver cancer caused by hepatitis virus is desired.

AIM (apoptosis inhibitor of macrophage) is a secretory blood protein with a molecular weight of about 50 kDa produced by tissue macrophages. AIM has a structure in which three scavenger recipient cysteine-rich (SRCR) domains, i.e., specific sequences containing many cysteine residues, are connected in tandem, and the cysteine residues are considered to be disulfide-bonded to each other in each domain to form a compact spherical three-dimensional structure.

AIM is known to have the characteristic of binding to various molecules such as lipopolysaccharide, IgM, complement regulatory factors, and fatty acid synthetases. Particularly, AIM is known to exist in the form of a complex with IgM in the blood. Since IgM is a huge protein complex exceeding 500 kDa, AIM does not pass through the glomerulus and transfer to urine as long as AIM is bound to IgM, and a high blood concentration is maintained. When dissociated from IgM, AIM is promptly excreted into urine. Therefore, most of AIM forms a complex with IgM in the blood and are rarely present in the blood in a free state rather than as a conjugate.

In recent years, it has been clarified that AIM is involved in the progression of pathological conditions in various diseases such as insulin resistance or arteriosclerosis. Non-Patent Document 3 describes that activation of apoptosis inhibitor of macrophage is a sensitive diagnostic marker for NASH-associated hepatocellular carcinoma. Non-Patent Document 4 relates to a proteomic study of differentially expressed proteins in Chronic Active Hepatitis, Cirrhosis, and HCC related to HCV infection in comparison with HBV Infection. Patent Document 1 discloses a method for detecting liver cancer comprising a step of detecting or quantifying free AIM in a biological sample derived from a subject. However, all of the liver cancers tested in Patent Document 1 are liver cancers caused by non-alcoholic fatty liver disease (NASH), and no association has been reported to date between free AIM and liver cancers caused by hepatitis virus.

### CITATION LIST

### PATENT LITERATURE

Patent Document 1: WO 2017/043617

### NON PATENT LITERATURE

Non-Patent Document 1: Pierangelo Fasani et al., HEPATOLOGY Vol. 29, No. 6, 1999
Non-Patent Document 2: Takeshi Okanoue et al., Rinsho Byori. 2016 May; 64(4): 472-479
Non-Patent Document 3: KOYAMA NORIYUKI et al., JOURNAL OF GASTROENTERLOGY vol. 53, No. 6, 2017
Non-Patent Document 4: SARVARI JAMAL et al., Hepatitis Monthly vol. 13, No. 7, 2013

### SUMMARY OF INVENTION

### TECHNICAL PROBLEM

An object of the present invention is to provide a method for distinguishing viral liver cancer from viral hepatitis or viral liver cirrhosis having excellent sensitivity and specificity, and a diagnostic agent kit for distinguishing viral liver cancer from viral hepatitis or viral liver cirrhosis having excellent sensitivity and specificity.

### SOLUTION TO PROBLEM distinguished from viral

As a result of intensive studie for solving the problems, the present inventor found that viral liver cancer can be distinguishied from viral hepatitis or viral liver cirrhosis with excellent sensitivity and specificity by measuring an amount of free AIM not bound to other substances in a biological sample and comparing the amount with a reference value, thereby completing the present invention. It is known that the association between a certain biomarker and liver cancer differs depending on a cause of liver cancer such as alcoholic, nonalcoholic fatty (NASH), or viral (Non-Patent Document 1). It is also reported that AFP serving as a biomarker for type C and type B viral liver cancer is not found to have high value in the diagnosis of liver cancer caused by NASH (Non-Patent Document 2). It is surprising that an association was found between an amount of free AIM in a biological sample and liver cancer caused by hepatitis virus.

Specifically, the present invention is as follows.
<1> A method for distinguishing viral liver cancer from viral hepatitis or viral liver cirrhosis in a subject comprising:
   measuring an amount of free AIM (apoptosis inhibitor of macrophage) in a biological sample from a subject
   comparing the measured amount of free AIM with a reference value
   where in free AIM means AIM not bound to other substances in the biological sample
   wherein the viral liver cancer is primary hepatocellular carcinoma caused by chronic hepatitis or cirrhosis due to persistent infection of the subject with hepatitis B virus and/or hepatitis C virus
   wherein the biological sample is a body fluid sample selected from blood, serum, plasma
   wherein an amount of free AIM in a biological sample from a subject is measured by bringing the biological sample into contact with an antibody specifically binding to the free AIM
<2> Use of a diagnostic agent kit for distinguishing viral liver cancer from viral hepatitis orviral liver cirrhosis in a subject, the diagnostic agent kit comprising:
   a reagent for measuring an amount of free AIM (apoptosis inhibitor of macrophage) in a bodyfluid sample
   where in free AIM means AIM not bound to other substances in the body fluid sample wherein the viral liver cancer is primary hepatocellular carcinoma caused by chronic hepatitis or cirrhosis due to persistent infection of the subject with hepatitis B virus and/or hepatitis C virus
   wherein the body fluid sample is selected from blood, serum, plasma
   wherein the diagnostic agent kit comprises an antibody specifically binding to the free AIM.
<3> The use of a diagnostic agent kit according to <2>, wherein the diagnostic agent kit comprises a solid phase on which the antibody specifically binding to the free AIM is immobilized and an anti-AIM antibody labeled with a labeling substance.

### ADVANTAGEOUS EFFECTS OF INVENTION

According to the present invention, viral liver cancer can be distinguished from viral hepatitis or viral liver cirrhosis with excellent sensitivity and specificity. According to the present invention, a diagnostic agent kit for distinguishing viral liver cancer from viral hepatitis or viral liver cirrhosis having excellent sensitivity and specificity can be provided.

### BRIEF DESCRIPTION OF DRAWINGS

[Fig. 1] Fig. 1 is a graph showing a comparison of amounts of free AIM in serum in type B viral hepatitis patients and type B viral liver cancer patients.
[Fig. 2] Fig. 2 is a graph showing a comparison of amounts of free AIM in serum in type C viral hepatitis patients and type C viral liver cancer patients.
[Fig. 3] Fig. 3 is a graph showing a comparison of abilities of AFP, PIVKA-II, and free AIM as biomarkers for hepatitis B viral liver cancer.
[Fig. 4] Fig. 4 is a graph showing a comparison of abilities of AFP, PIVKA-II, and free AIM as biomarkers for hepatitis C viral liver cancer.

### DESCRIPTION OF EMBODIMENTS

### [1] Method for Detecting Viral Liver Cancer

### (Biological sample from subject)

Examples of a biological sample analyzable in the present invention include body fluids derived from living bodies (organisms). The biological sample in the present invention is blood, serum, or plasma, preferably blood, serum, or plasma of a subject possibly having viral liver cancer. Examples of the living body or the subject include humans or animals (e.g., mice, guinea pigs, rats, monkeys, dogs, cats, hamsters, horses, bovines, and pigs), and are preferably humans. The biological sample from the subject may be collected or prepared at the time of implementation of the present invention or may preliminarily be collected or prepared and stored. The person preparing the sample and the person measuring an amount of free AIM in the sample may be different. The biological sample can be an in vivo or in vitro sample. The biological sample can be a biological sample of a subject possibly having viral liver cancer.

### (AIM)

AIM (apoptosis inhibitor of macrophage) is a secretory blood protein with a molecular weight of about 50 kDa produced by tissue macrophages. AIM has a structure in which three scavenger recipient cysteine-rich (SRCR) domains, i.e., specific sequences containing many cysteine residues, are connected in tandem, and the cysteine residues are considered to be disulfide-bonded to each other in each domain to form a compact spherical three-dimensional structure.

### (Free AIM)

In this description, the "free AIM" means AIM existing in a free state without being bound to other substances such as lipopolysaccharide or IgM. On the other hand, in this description, AIM bound to other substances such as lipopolysaccharide or IgM and existing in a state of a complex may be referred to as complex AIM. The free AIM can be human free AIM.

### (Method for Measuring Free AIM)

The method for measuring or quantifying free AIM is an immunological analysis method using an anti-AIM antibody. The term "anti-AIM antibody" means an antibody capable of reacting with the free AIM.

Examples of the immunoassay method using an anti-AIM antibody include, but not limited to, electrochemiluminescence immunoassay (ECL method), ELISA, enzyme immunoassay, immunohistological staining, surface plasmon resonance, latex agglutination immunoassay, chemiluminescence immunoassay, a fluorescent antibody method, radioimmunassay, an immunoprecipitation method, a Western Blot method, immunochromatography, high performance liquid chromatography (HPLC), etc.

The anti-AIM antibody can be prepared as either a monoclonal antibody or a polyclonal antibody according to a known method. The monoclonal antibody can be obtained by, for example, isolating spleen cells or lymph node cells, which are antibody-producing cells, from a non-human mammal immunized with the free AIM or a free AIM fragment, by fusing the cells with a myeloma-derived cell line having a high proliferative capacity to produce a hybridoma, and by purifying an antibody produced by this hybridoma. The polyclonal antibody can be obtained from the sera of an animal immunized with the free AIM or a free AIM fragment. The free AIM fragment is a partial peptide of the free AIM, and the antibody binding to the free AIM fragment recognizes the free AIM. Examples of immunogens include, but not limited to, the free AIM or free AIM fragments of primates such as humans and monkeys, rodents such as rats and mice, dogs, cats, horses, sheep, and pigs.

The anti-AIM antibody can be a whole antibody molecule as well as a fragment of an antibody having an antigen-antibody reaction activity and can be an antibody obtained through an immunization step of an animal as described above or obtained by a gene recombination technique, or a chimeric antibody. The fragment of the antibody is preferably a functional fragment; examples thereof include F(ab')₂, Fab', scFv, etc.; and these fragments can be produced by processing the antibody obtained as described above with a proteolytic enzyme (e.g., pepsin or papain), or by cloning of DNA of the antibody and expression in a culture system using Escherichia coli or yeast.

The anti-AIM antibody means an antibody reacting with AIM. The present invention employs an antibody specifically reacting with free AIM. "Specifically reacting with free AIM" means that the antibody reacts with free AIM and does not substantially react with other substances, especially the complex AIM. The meaning of "not substantially reacting" will be described later. The anti-AIM antibody or the antibody specifically reacting with free AIM used in the present invention is preferably a monoclonal antibody.

Although "reacting" with free AIM, "recognizing" free AIM, and "binding" to free AIM are synonymously used in this description, they must be construed in the broadest sense without being limited to these exemplifications. Whether an antibody "reacts" with an antigen (compound) such as free AIM can be confirmed by an antigen solid phase ELISA method, a competitive ELISA method, a sandwich ELISA method, etc. as well as by a method (SPR method) using the principle of surface plasmon resonance etc. The SPR method can be performed by using devices, sensors, and reagents commercially available under the name of Biacore (registered trademark).

Stating that the antibody used "does not react with" a certain compound means that the antibody used in the present invention does not substantially react with a certain compound, while stating "not substantially reacting" means that enhanced reactivity of the antibody used in the present invention is not recognized when Biacore (registered trademark) T100 or T200 is used for immobilizing the antibody of the present invention to perform measurement based on the SPR method, for example. Specifically, this means that the reactivity between the antibody and the compound is not significantly different from the reactivity of a control (with no compound added). Obviously, it can be confirmed that the antibody "does not substantially react" with the compound by a method or means well known to those skilled in the art other than the SPR method.

In this description, an "insoluble carrier" may be represented as a "solid phase", and physically or chemically supporting an antigen or antibody with an insoluble carrier or the supporting state may be represented as "immobilizing", "immobilized", or "solid phased". The term "analysis", "detection", or "measurement" must be construed in the broadest sense, including the existence proof and/or the quantification of free AIM and must not be construed in a limited manner in any sense.

By using a labeled antibody (secondary antibody) that can bind to the antibody used, an amount of antibody bound to the free AIM can be measured, and the free AIM in the biological sample can thereby be detected. Examples of a labeling substance for producing the labeled antibody include enzymes, fluorescent substances, chemical luminescent substances, biotin, avidin, radioisotopes, colloidal gold particles, or colored latex. Although those skilled in the art can appropriately select an immunological analysis method depending on an antibody and a labeling substance used, the electrochemiluminescence immunoassay (ECL method) is preferably used since the experimental system can easily be constructed.

The electrochemiluminescence immunoassay (ECL method) means a method of calculating an amount of an analyte by causing a labeling substance to emit light by an electrochemical stimulus and detecting an amount of luminescence. In the electrochemiluminescence immunoassay (ECL method), a ruthenium complex can be used as a labeling substance. The amount of luminescence of this ruthenium complex can be detected by disposing an electrode on a solid phase (microplate or beads etc.) and causing an electrochemical stimulus on the electrode.

Electrochemiluminescent immunoassay (ECL method) can be performed by using an anti-AIM antibody as a solid phase antibody and another antibody binding to free AIM as a detection antibody (labeled antibody). An antibody specifically binding to the free AIM is used as the solid phase antibody and/or the labeled antibody, and an antibody specifically binding to free AIM is preferably used as the solid phase antibody. When the solid phase antibody and the labeled antibody are used and beads and a ruthenium complex are used as a solid phase and a label, respectively, the measurement principle is as follows. The following description describes the measurement principle in an embodiment of the present invention and does not limit the present invention at all.
1. When the beads having the anti-AIM antibody bound thereto are reacted with a sample, the free AIM in the sample binds to the antibody bound to the beads.
2. After washing the beads, a ruthenium-labeled antibody (an antibody having a recognition epitope different from 1.) is reacted with the free AIM bound to the beads and is bound in a sandwich shape.
3. After washing the beads, when electrical energy is applied on the electrode, the ruthenium complex emits light depending on an amount of the ruthenium-labeled antibody bound to the beads via the free AIM. By measuring this amount of luminescence, the free AIM in the sample can accurately be measured.

Among the immunological analysis methods, the ELISA method using an enzyme label is also preferable since a target can easily and quickly be measured. In the case of sandwich ELISA, an insoluble carrier having an anti-AIM antibody immobilized thereon and an anti-AIM antibody labeled with a labeling substance and having an epitope different from the immobilized antibody can be used. In this case, the insoluble carrier is preferably a plate (immunoplate), and the labeling substance can appropriately be selected and used. The antibody to be bound to the insoluble carrier and/or the antibody labeled with the labeling substance is an antibody specifically binding to the free AIM, and it is preferable that the antibody specifically binding to the free AIM is used as the solid phase antibody.

The antibody immobilized on the insoluble carrier captures the free AIM in the sample and forms an antibody-free AIM complex on the insoluble carrier. The antibody labeled with the labeling substance binds to the captured free AIM to form a sandwich with the antibody-free AIM complex described above. The free AIM in the sample can be measured by measuring an amount of the labeling substance by a method corresponding to the labeling substance. For specific methods, such as a method for immobilizing the antibody on the insoluble membrane and a method for binding the antibody to the labeling substance, the methods well known to those skilled in the art can be used without limitation.

A latex immunoagglutination method (hereinafter also referred to as an LTIA method) is a typical particle agglutination immunoassay and is also preferable as an immunological analysis method. In the LTIA method, latex particles carrying an antibody to a target component are used, and a degree of aggregation (turbidity) of the latex particles caused by binding between an antigen that is the target component and the antibody-supporting latex particles forming an antigen-antibody complex is detected by optical means (e.g., a turbidimetric method for measuring transmitted light, a turbidity method for measuring scattered light), so that the target component can be analyzed. In the immunological analysis method of the present invention, latex particles carrying the anti-AIM antibody are used, and a degree of aggregation of the latex particles caused by binding between free AIM that is the target component and the antibody-supporting latex particles forming an antigen-antibody complex can be detected by optical means. When LTIA is used as the immunological analysis method, at least one antibody specifically binding to free AIM is used.

In the present invention, a commercially available kit capable of specifically detecting free AIM may be used for measuring an amount of free AIM. Examples of the commercially available kit capable of specifically detecting free AIM include, but not limited to, Human AIM ELISA kit (CY-8080; Circulex).

### (Viral Liver Cancer)

In this description, viral liver cancer means primary hepatocellular carcinoma caused by chronic hepatitis and cirrhosis due to persistent infection with a hepatitis virus. The viral liver cancer distinguished from viral hepatitis or viral liver cirrhosis by the present invention is a viral liver cancer caused by HBV (hepatitis B virus) and/or HCV (hepatitis C virus).

### (Reference Value)

The method for detecting viral liver cancer of the present invention includes comparing a measured amount of free AIM with a reference value. In the method for distinguishing viral liver cancer from viral hepatitis or viral liver cirrhosis of the present invention, viral liver cancer can be distinguished by using as an index the fact that an amount of free AIM in a subject is higher than an amount of free AIM in a healthy subject group. Specifically, for example, viral liver cancer can be distinguished when the amount of free AIM of the subj ect becomes equal to or greater than a threshold value (reference value) for determination with respect to the healthy subject group.

A range of numerical values can be used as the reference value. When it is diagnosed whether a person has viral liver cancer, ranges of amounts of free AIM are measured in advance in biological samples of subjects having been diagnosed as having viral liver cancer and subjects having been diagnosed as not having viral liver cancer, and if an amount of free AIM in a biological sample of a subject falls within the range of the amount of free AIM in the biological samples of healthy subjects, this subject is unlikely to have viral liver cancer, while if the amount of free AIM falls within the range of the amount of free AIM in the biological samples of the subjects having viral liver cancer, the subject is likely to have viral liver cancer.

Although the threshold value (reference value) for determination is expected to change depending on various conditions such as underlying disease, gender, and age, those skilled in the art can appropriately select a proper population corresponding to the subject and statistically process data obtained from the population to determine a normal value range or the threshold value for determination. For example, the reference value can be 0.1 µg/mL, 0.2 µg/mL, 0.3 µg/mL, 0.4 µg/mL, 0.5 µg/mL, 0.6 µg/mL, and 0.7 µg/mL, 0.8 µg/mL, 0.9 µg/mL, 1.0 µg/mL, 1.1 µg/mL, 1.2 µg/mL, 1.3 µg/mL, 1.4 µg/mL, 1.5 µg/mL, 1.6 µg/mL, 1.7 µg/mL, 1.8 µg/mL, 19 µg/mL, 2.0 µg/mL, 2.1 µg/mL, 2.2 µg/mL, 2.3 µg/mL, 2. 4 µg/mL, 2.5 µg/mL, 2.6 µg/mL, 2.7 µg/mL, 2.8 µg/mL, 2.9 µg/mL, 3.0 µg/mL, 3.1 µg/mL, 3.2 µg/It can be mL, 3.3 µg/mL, 3.4 µg/mL, or 3.5 µg/mL as a value in human serum. For example, as shown in Examples described later, the reference value can be 2.2 µg/mL as a value in human serum.

The method for distinguishing viral liver cancer from viral hepatitis or viral liver cirrhosis of the present invention can be a method for assisting diagnosis of viral liver cancer including measuring an amount of free AIM in a biological sample from a subject and comparing the measured amount of free AIM with a reference value. The method for distinction of the present invention can further include implementing another method for detecting viral liver cancer in a patient and/or treating viral liver cancer based on a result as needed.

The method for distinguishing viral liver cancer from viral hepatitis or viral liver cirrhosis of the present invention can also determine whether viral hepatitis or cirrhosis in a subject has progressed to viral liver cancer. In this case, the amount of free AIM in the subject at a certain time point can be used as a threshold value (reference value) for determination. After a certain period (e.g., after 1, 3, 6, or 12 months), the amount of free AIM in this subject is measured again, and if the amount of free AIM is significantly higher than the previous measurement value, it can be determined that viral hepatitis or cirrhosis has progressed to viral liver cancer. Conversely, if the amount of free AIM is not changed as compared to the previously measurement values, it can be determined that viral hepatitis or cirrhosis has not progressed to viral liver cancer.

### [2] Use of a diagnostic agent kit

The invention also provides use of a diagnostic kit in accordance with independent claim 2. Kits *per se* are not claimed.

The diagnostic kit preferably includes a solid phase on which a first anti-AIM antibody is immobilized, and a second anti-AIM antibody labeled with a labeling substance. The first anti-AIM antibody and the second anti-AIM antibody recognize different epitopes. When the ECL method is used, the diagnostic agent kit can include a solid phase on which the anti-AIM antibody is immobilized, and an anti-AIM antibody labeled with an electrochemically luminescent substance such as a ruthenium complex. The antibody immobilized on the solid phase and/or the antibody labeled with the labeling substance is an antibody specifically binding to the free AIM, and it is more preferable that the antibody specifically binding to the free AIM is used as the antibody immobilized on the solid phase. For example, in the kit using microbeads as the solid phase, a biological sample is added to and reacted with the microbeads on which the anti-AIM antibody is immobilized, and the sample is then removed and washed. Subsequently, an anti-AIM antibody recognizing another epitope labeled with an electrochemically luminescent substance is added and reacted. After washing the microbeads, electric energy is applied for luminescence, and an amount of luminescence of the labeling substance can be measured to obtain a free AIM concentration.

When the sandwich ELISA method is used, the diagnostic agent kit at least includes an insoluble carrier on which the first anti-AIM antibody (solid phase antibody) is immobilized, and a second anti-AIM antibody (labeled antibody) labeled with a labeling substance and binding to the free AIM. The first anti-AIM antibody and the second anti-AIM antibody recognize different epitopes. In such a kit, first, a biological sample is added to the insoluble carrier on which the first anti-AIM antibody is immobilized, and is then incubated, and the sample is removed and washed. The labeled antibody is added and then incubated, and a substrate is added for coloring. The free AIM concentration can be determined by measuring the coloring with a plate reader etc. The antibody immobilized on the insoluble carrier and/or the antibody labeled with the labeling substance is an antibody specifically binding to the free AIM, and it is more preferable that the antibody specifically binding to the free AIM is used as the antibody immobilized on the insoluble carrier.

When the LTIA method is used, the diagnostic agent kit includes at least the following (1) and (2):
(1) latex particles on which the first anti-AIM antibody is immobilized; and
(2) latex particles on which the second anti-AIM antibody recognizing an epitope different from the first anti-AIM antibody is immobilized.

In such a kit, the first anti-AIM antibody and the second anti-AIM antibody aggregate via free AIM. The free AIM concentration in the biological sample can be obtained by detecting a degree of aggregation by using optical means. At least one of the first anti-AIM antibody and the second anti-AIM antibody is an antibody specifically binding to the free AIM.

The present invention will hereinafter specifically be described with examples; however, these examples do not limit the present invention.

### EXAMPLES

### 1. Production of Mouse Anti-Human AIM Monoclonal Antibody

An antibody No. 11 and an antibody No. 12 were mouse anti-human AIM monoclonal antibodies and were obtained by the following procedure.

Emulsion was produced by mixing full-length human rAIM (2 mg/ml) as an antigen with an equal amount of TiterMax Gold (G-1 Funakoshi). Two 8-week-old female Balb/c mice (Charles River Laboratories) were used as immunized animals, and 50 µL of an antigen solution was administered to the sole of the hind foot. The same administration was performed 2 weeks later, and after another 2 weeks or more, 50 µg of the antigen solution was administered to the sole of the hind foot to prepare for cell fusion performed 3 days later.

Mouse P3U1 was used for myeloma cells, and a medium used for growth culture was obtained by adding glutamine and pyruvic acid to RPMI1640 (11875-119 GIBCO) and adding FBS (S1560 BWT) at 10 %. Penicillin and streptomycin were added as antibiotics in appropriate amounts.

Popliteal lymph nodes were aseptically removed from the mice after cardiac blood was collected under anesthesia and were placed on a beaker with #200 mesh and pressed with a silicon rod to prepare a cell suspension. The cells were centrifugally washed twice in RPMI 1640 and then the number of cells was counted. Myeloma cells in the logarithmic growth phase were collected by centrifugation, washed, and then prepared so that the ratio of lymphocytes to myeloma cells was 5:1, and mixing centrifugation was performed. Cell fusion was performed by using PEG1500 (783641 Roche). Specifically, after a cell pellet was reacted with 1 mL of PEG solution for 3 minutes, then diluted in stages, and washed by centrifugation, a medium was added, and 200 µL was placed in each of 15 96-well plates for 1 week of culture. For the medium, a HAT supplement (21060-017 GIBCO) was added to a medium for myeloma cells to adjust the FBS concentration to 15 %.

After the cryopreserved cells were thawed and proliferation culture was performed, 1×10⁷ cells were administered to the abdominal cavity of a nude mouse (BALB/cAJcl-nu/nu Nippon Claire) to which 0.5 ml of pristane (42-002 Cosmo Bio) was intraperitoneally administered 1 week or more before, and after about 2 weeks, 4 to 12 ml of ascites was obtained. After removing a solid matter by centrifugation, the ascites was cryopreserved. Subsequently, antibodies were purified from the cryopreserved ascites to obtain the antibody No. 11 and the antibody No. 12.

### 2. Production of Antibody-Bound Magnetic Beads

1) The absorbance of the antibody No. 12 dialyzed with 150 mM potassium phosphate buffer (pH 7.8) was measured and adjusted to Abs 0.5 by using the same buffer solution.
2) With the buffer solution, 1 mL (30 mg/mL) of Dynabeads M-450 Epoxy manufactured by Dynamic Biotech was washed 3 times, and 1 mL of the antibody solution of 1) was added. Rotary stirring was performed at 25 °C for 18 hours or more.
3) The beads were washed twice with a bead blocking buffer [50 mM Tris, 150 mM NaCl, 0.1 % BSA (fatty acid free), 0.1 % NaN₃, pH 7.8]. The antibody remaining in the solution and not bound to the beads was removed by removing the buffer solution by washing. Subsequently, 1 mL of the bead blocking buffer was added and stirred, and rotary stirring was performed at 25 °C for 18 hours or more.
4) After washing the beads twice with the bead blocking buffer, 1 mL of the bead blocking buffer was added and stirred.

These were used as anti-AIM antibody-bound magnetic beads and stored at 4 °C until use.

### 3. Preparation of Ruthenium-Labeled Antibody

1) To 312.5 µL of an antibody No. 11 solution dialyzed with 150 mM potassium phosphate buffer (pH 7.8), 14.1 µL of 10 mg/mL ruthenium complex (Origin Tag-NHS ESTER manufactured by IGEN) was added, and the solution was stirred for 30 minutes. Subsequently, 50 µL of 2M glycine was added, and the solution was stirred for 20 minutes.
2) A ruthenium complex-labeled antibody was applied to gel filtration column chromatography (SEphadex G-25 manufactured by GE Healthcare Bioscience) packed in a glass tube with a diameter of 1 cm and a height of 30 cm to isolate and purify the non-labeling ruthenium complex and the ruthenium complex-labeled antibody. Elution was performed with 10 mM potassium phosphate buffer (pH 6.0).

### 4. Quantification of Free AIM in Serum

1) Each of patient's serum specimens (19 specimens of viral hepatitis B, 27 specimens of type B viral liver cancer, 55 specimens of viral hepatitis C, 45 specimens of type C viral liver) was diluted in a reaction solution [50 mM HEEPS, 50 mM NaCl, 0.05 % Tween 20, 1 mM EDT-4Na, 0.5 % BSA, 0.1 % NaN₃, 100 µg/mL Mouse IgG, pH 7.8] to 1/10 to produce a specimen diluted solution. Subsequently, 100 µL of the reaction solution was placed in a reaction tube, and 2 µL of the specimen diluted solution was added.
2) To the solution, 25 µL of antibody No. 12-bound magnetic beads diluted to a concentration of 0.5 mg/mL with a bead diluent [50 mM HEPES, 100 mM NaCl, 0.1 % Tween 20, 1 mM EDT-4Na, 0.5 % BSA (fatty acid free), 0.1 % NaN₃, pH 7.8] was added and reacted at 30 °C for 9 minutes (first reaction).

Subsequently, the magnetic beads were trapped with a magnet, the liquid in the reaction tube was extracted, and the magnetic beads were washed twice with 350 µL of cleaning liquid [50 mmol/L Tris HCl, 0.01 % (W/V) Tween 20, 0.15 mol/L NaCl, pH 7.5] to remove non-specific binding substances other than the antigen-antibody reaction (BF separation).
3) Subsequently, 200 µL of the ruthenium-labeled antibody No. 11 diluted with a dilute solution for ruthenium [50 mM HEPES, 50 mM NaCl, 0.05 % Tween 20, 1 mM EDT-4Na, 0.5 % BSA, 0.1 % NaN₃, 100 µg/mL mouse IgG, pH 7.8] to a concentration of 0.6 µg/mL was added and reacted at 30 °C for 9 minutes (second reaction).

The magnetic beads after the reaction were trapped with a magnet, the liquid in the reaction tube was extracted, and the magnetic beads were washed twice with 350 µL of the washing solution to remove non-specific binding substances other than the antigen-antibody reaction (BF separation). 4) Subsequently, 300 µL of tripropylamine was placed in the reaction tube and mixed with magnetic beads.

By applying electrical energy in this state, the ruthenium complex emitted light, and the emission intensity was detected by a detector.

After the operation of adding the magnetic beads to the reaction tube, this was performed on Picolumi III, which is an automatic ruthenium complex luminescence measuring machine.

### 5. Distribution and ROC Analysis of Free AIM values in viral hepatitis patients and liver cancer patients

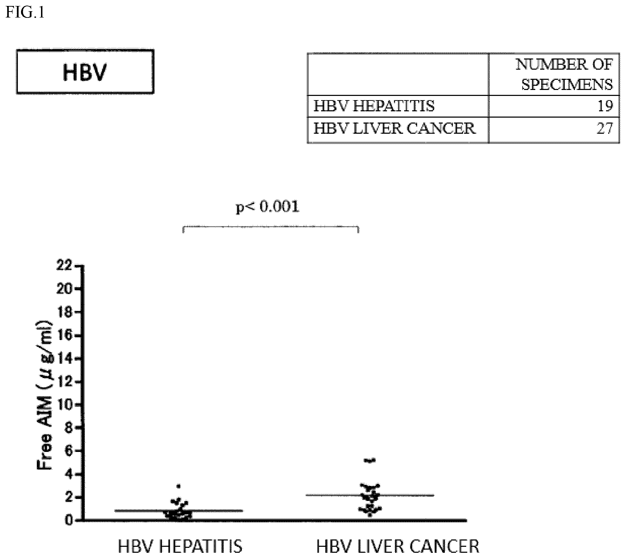

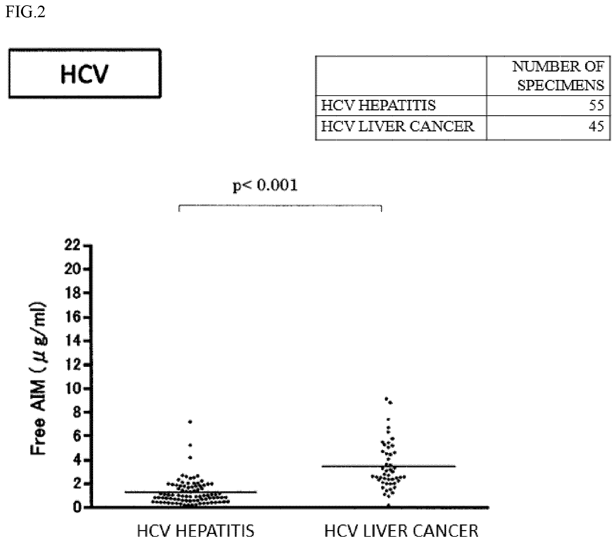

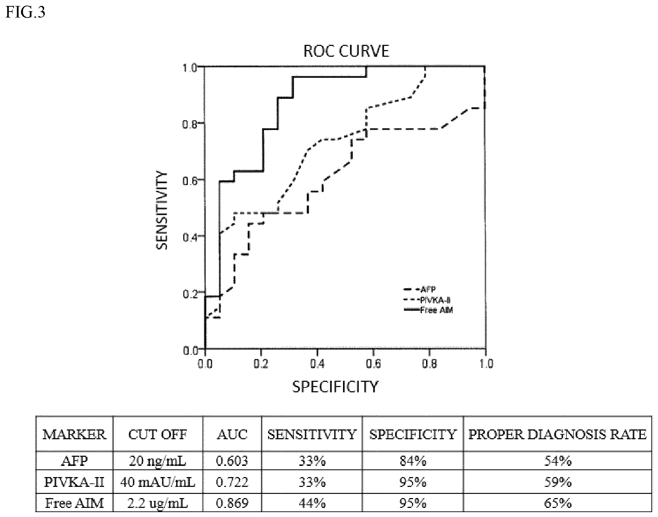

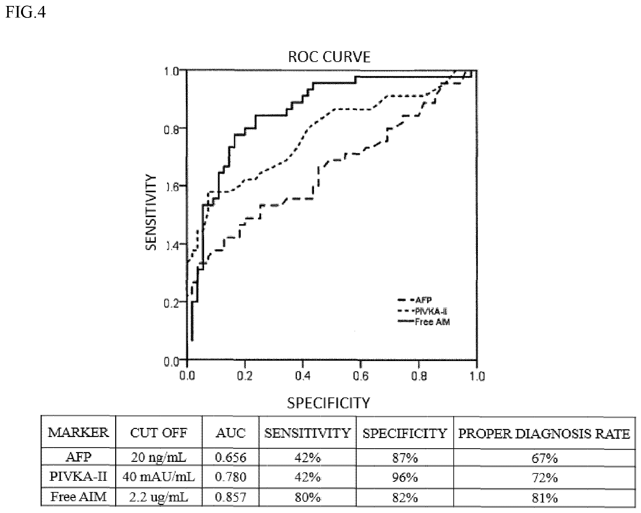

1) Distribution of free AIM values was confirmed in 19 specimens of viral hepatitis B and 27 specimens of type B viral liver cancer, and distribution of free AIM values was also confirmed in 55 specimens of viral hepatitis C and 45 specimens of type C viral liver cancer. The results are shown in Figs. 1 and 2.
2) As shown in Fig. 1, a significant difference was observed in the amount of free AIM between HBV hepatitis patients and HBV liver cancer patients. Therefore, it was found that by measuring the amount of free AIM, a type B viral hepatitis patient can be distinguished from a type B viral liver cancer patient. As shown in Fig. 2, a significant difference was observed in the amount of free AIM between HCV hepatitis patients and HCV liver cancer patients. Therefore, it was found that by measuring the amount of free AIM, a type C viral hepatitis patient can be distinguished from a type C viral liver cancer patient.
3) For the free AIM values of the hepatitis and liver cancer specimens described above, ROC analysis was performed by using IBM SPSS Statistics ver. 24 to calculate AUC. ROC analysis was also performed for existing tumor markers (AFP, PIVKA-II) and compared with the free AIM. The results are shown in Figs. 3 and 4.
4) In the detection of HBV, the free AIM was superior to both AFP and PIVKA-II in AUC, sensitivity, and proper diagnosis rate. In terms of specificity, the free AIM had the same value as PIVKA-II and was superior to AFP.

In the detection of HCV, the free AIM was superior to both AFP and PIVKA-II in AUC, sensitivity, and proper diagnosis rate. In terms of specificity, the free AIM was lower than PIVKA-II and AFP.

### INDUSTRIAL APPLICABILITY

According to the present invention, viral liver cancer can be distinguished from viral hepatitis or viral liver cirrhosis with excellent sensitivity and specificity. According to the present invention, use of a diagnostic agent kit for distinguishing viral liver cancer from viral hepatitis or viral liver cirrhosis having excellent sensitivity and specificity can be provided.

## Claims

1. A method for distinguishing viral liver cancer from viral hepatitis or viral liver cirrhosis in a subject comprising:
measuring an amount of free AIM (apoptosis inhibitor of macrophage) in a biological sample from a subject
comparing the measured amount of free AIM with a reference value
wherein free AIM means AIM not bound to other substances in the biological sample
wherein the viral liver cancer is primary hepatocellular carcinoma caused by chronic hepatitis or cirrhosis due to persistent infection of the subject with hepatitis B virus and/or hepatitis C virus
wherein the biological sample is a body fluid sample selected from blood, serum, plasma
wherein an amount of free AIM in a biological sample from a subject is measured by bringing the biological sample into contact with an antibody specifically binding to the free AIM

2. Use of a diagnostic agent kit for distinguishing viral liver cancer from viral hepatitis or viral liver cirrhosis in a subject, the diagnostic agent kit comprising:
a reagent for measuring an amount of free AIM (apoptosis inhibitor of macrophage) in a body fluid sample
wherein free AIM means AIM not bound to other substances in the body fluid sample
wherein the viral liver cancer is primary hepatocellular carcinoma caused by chronic hepatitis or cirrhosis due to persistent infection of the subject with hepatitis B virus and/or hepatitis C virus
wherein the body fluid sample is selected from blood, serum, plasma
wherein the diagnostic agent kit comprises an antibody specifically binding to the free AIM.

3. The use of a diagnostic agent kit according to claim 2, wherein the diagnostic agent kit comprises a solid phase on which the antibody specifically binding to the free AIM is immobilized and an anti-AIM antibody labeled with a labeling substance.

## Patentansprüche

1. Verfahren zum Unterscheiden von viralem Leberkrebs von viraler Hepatitis oder viraler Leberzirrhose in einem Subjekt, umfassend:
Messen einer Menge an freiem AIM (Apoptoseinhibitor von Makrophagen) in einer biologischen Probe von einem Subjekt
Vergleichen der gemessenen Menge an freiem AIM mit einem Referenzwert
wobei freies AIM bedeutet, dass AIM nicht an andere Substanzen in der biologischen Probe gebunden ist
wobei der virale Leberkrebs ein primäres hepatozelluläres Karzinom ist, das durch chronische Hepatitis oder Zirrhose aufgrund einer anhaltenden Infektion des Patienten mit Hepatitis B Virus und/oder Hepatitis C Virus verursacht wird
wobei die biologische Probe eine Körperflüssigkeitsprobe ist, ausgewählt aus Blut, Serum, Plasma,
wobei eine Menge an freiem AIM in einer biologischen Probe eines Subjekts gemessen wird, indem die biologische Probe mit einem Antikörper in Kontakt gebracht wird, der spezifisch an das freie AIM bindet.

2. Verwendung eines Diagnostik-Kits zum Unterscheiden von viralem Leberkrebs von viraler Hepatitis oder viraler Leberzirrhose in einem Subjekt, wobei das Diagnostik-Kit umfasst:
ein Reagenz zum Messen der Menge an freiem AIM (Apoptoseinhibitor von Makrophagen) in einer Körperflüssigkeitsprobe
wobei freies AIM bedeutet, dass AIM nicht an andere Substanzen in der Körperflüssigkeitsprobe gebunden ist
wobei der virale Leberkrebs ein primäres hepatozelluläres Karzinom ist, verursacht durch chronische Hepatitis oder Zirrhose aufgrund einer anhaltenden Infektion des Patienten mit dem Hepatitis B Virus und/oder Hepatitis C Virus
wobei die Körperflüssigkeitsprobe ausgewählt ist aus Blut, Serum, Plasma
wobei das Diagnostik-Kit einen Antikörper umfasst, der spezifisch an das freie AIM bindet.

3. Verwendung eines Diagnostik-Kits gemäß Anspruch 2, wobei das Diagnostik-Kit eine feste Phase, auf der der spezifisch an das freie AIM bindende Antikörper immobilisiert ist, und einen mit einer Markierungssubstanz markierten Anti-AIM-Antikörper umfasst.

## Revendications

1. Procédé permettant de distinguer le cancer viral du foie de l'hépatite virale ou de la cirrhose virale du foie chez un sujet comprenant :
la mesure d'une quantité d'AIM (inhibiteur d'apoptose des macrophages) libre dans un échantillon biologique d'un sujet
la comparaison de la quantité mesurée de AIM libre avec une valeur de référence
dans lequel AIM libre signifie AIM non lié à d'autres substances dans l'échantillon biologique
dans lequel le cancer viral du foie est un carcinome hépatocellulaire primaire causé par une hépatite chronique ou une cirrhose due à une infection persistante du sujet par le virus de l'hépatite B et/ou le virus de l'hépatite C
dans lequel l'échantillon biologique est un échantillon de fluide corporel choisi parmi le sang, le sérum, le plasma
dans lequel une quantité d'AIM libre dans un échantillon biologique provenant d'un sujet est mesurée en mettant l'échantillon biologique en contact avec un anticorps se liant spécifiquement à l'AIM libre

2. Utilisation d'un kit d'agent diagnostique pour distinguer le cancer viral du foie de l'hépatite virale ou de la cirrhose virale du foie chez un sujet, le kit d'agent diagnostique comprenant :
un réactif pour mesurer une quantité d'AIM (inhibiteur d'apoptose des macrophages) libre dans un échantillon de fluide corporel
dans laquelle AIM libre signifie AIM non lié à d'autres substances dans l'échantillon de fluide corporel
dans laquelle le cancer viral du foie est un carcinome hépatocellulaire primaire causé par une hépatite chronique ou une cirrhose due à une infection persistante du sujet par le virus de l'hépatite B et/ou le virus de l'hépatite C
dans laquelle l'échantillon de fluide corporel est choisi parmi le sang, le sérum, le plasma
dans laquelle le kit d'agent diagnostique comprend un anticorps se liant spécifiquement à l'AIM libre.

3. Utilisation d'un kit d'agent diagnostique selon la revendication 2, dans laquelle le kit d'agent diagnostique comprend une phase solide sur laquelle l'anticorps se liant spécifiquement à l'AIM libre est immobilisé et un anticorps anti-AIM marqué avec une substance de marquage.
